# EUROPEAN PATENT SPECIFICATION

(11) **EP 3 870 268 B1**
(45) Date of publication and mention of the grant of the patent: **19.10.2022**
(21) Application number: 19804953.8
(22) Date of filing: 24.10.2019
(51) Int. Cl.: A61N 1/05, A61N 1/362

(54) **CONFIGURATIONS AND CONSTRUCTIONS FOR ACTIVE FIXATION IMPLANTABLE MEDICAL ELECTRICAL LEADS**
KONFIGURATIONEN UND KONSTRUKTIONEN ZUR AKTIVEN FIXIERUNG VON IMPLANTIERBAREN MEDIZINISCHEN ELEKTRODENLEITUNGEN
CONFIGURATIONS ET CONSTRUCTIONS POUR FILS ÉLECTRIQUES MÉDICAUX IMPLANTABLES À FIXATION ACTIVE

(30) Priority: 26.10.2018 US 201862751315 P; 23.10.2019 US 201916661605
(43) Date of publication of application: 01.09.2021
(73) Proprietor: Medtronic, Inc., Minneapolis, MN 55432 (US)
(72) Inventor: CLEMENS, William J., Minneapolis, MN 55432 (US); LEE, Vania X., Minneapolis, MN 55432 (US); PISTELLA, Maggie J., Minneapolis, MN 55432 (US)
(74) Representative: Zimmermann & Partner Patentanwälte mbB
(86) International application number: PCT/US2019/057780
(87) International publication number: WO 2020/086798

(56) References cited:
- US-A1- 2007 250 144
- US-A1- 2014 046 389
- US-A1- 2016 339 235

## Description

### FIELD OF THE DISCLOSURE

The present disclosure pertains to implantable medical electrical leads, and more particularly to configurations and constructions of leads that include active fixation elements.

### BACKGROUND

An implantable medical electrical system of the type suited for cardiac rhythm management typically includes a pulse generator device to which at least one flexible elongate lead is coupled to deliver stimulation therapy from the pulse generator to the heart of a patient. The pulse generator device may be implanted in a subcutaneous pocket, remote from the heart, with the lead extending from the device to a cardiac implant site, either endocardial or epicardial, where one or more electrodes of the lead are secured to deliver the stimulation therapy.

With the advent of left heart pacing, for example, to alleviate heart failure, leads may be implanted within the coronary venous system so that electrodes thereof are positioned at left ventricular pacing sites, which are typically located in proximity to the base of the left ventricle. Leads configured for implantation within the coronary venous system may include one or more fixation elements to secure the electrodes thereof at the pacing sites. There is still a need for new constructions and configurations of active fixation implantable medical electrical leads to meet the demand for therapy delivery via left heart pacing. A active fixation lead is described in US 2016/339235, the lead comprising a connector terminal, a grip sleeve, two electrodes connected via two elongated conductors to two connector terminal contacts, the lead further comprising a fixation element with a piercing distal tip and an elongated outer insulation tube having at the distal segment a larger diameter than at the proximal segment.

### SUMMARY

According to embodiments disclosed herein, an implantable medical electrical lead includes a plurality of longitudinally spaced electrodes and a fixation element located between a proximal-most pair of adjacent electrodes of the plurality of electrodes; the fixation element includes a wire wound into a tissue engaging helix that has one or more exposed turns extending around and spaced laterally from an outer surface of the lead that extends between the proximal-most pair of adjacent electrodes. According to some embodiments, a piercing tip of the fixation element wire, which terminates the one or more exposed turns of the helix and is also spaced laterally from the lead outer surface, is centered between the proximal-most pair of adjacent electrodes. A connector terminal grip sleeve of some lead embodiments may be gripped by an operator to apply a torque which is transferred along the lead and to the fixation element, thereby engaging the helix thereof with tissue, for example, to secure the lead electrodes within the coronary venous system of a patient. According to some embodiments, the lead includes means for limiting the amount of torque that can be transferred from the connector terminal grip sleeve to the fixation element. In some embodiments, the torque limiting means may be an external contour of the lead along a length thereof that extends between the connector terminal grip sleeve and the fixation element, while, according to some alternate embodiments, the torque limiting means may be an accessory sleeve mounted around the connector terminal grip sleeve. The plurality of lead electrodes may further include a distal-most pair of adjacent electrodes, between which the lead extends in pre-set a curvature.

According to some constructions, any of the lead configurations disclosed herein may have an outer insulation formed by a plurality of elongate outer insulation tubes. An outer insulation tube formed to extend between the proximal-most pair of adjacent electrodes may have the aforementioned fixation element secured thereto so that the piercing distal tip of the fixation element is centered between proximal and distal ends of the tube; the proximal and distal ends of the tube may be secured to a corresponding electrode of the proximal-most pair either before or after securing the fixation element to the tube. Another outer insulation tube that has a length to extend from the connector terminal to a proximal electrode of the proximal-most pair may be ablated to form the aforementioned external contour of the lead. And yet another outer insulation tube that has a length to extend between the distal-most pair of adjacent electrodes may be pre-formed into a bend and thus impart the aforementioned pre-set curvature to the lead.

### BRIEF DESCRIPTION OF THE DRAWINGS

The following drawings are illustrative of particular embodiments of the present disclosure and therefore do not limit the scope of the invention as defined in independent claim 1. The drawings are not to scale (unless so stated) and are intended for use in conjunction with the explanations in the following detailed description.

Embodiments will hereinafter be described in conjunction with the appended drawings wherein like numerals denote like elements, and:
Figure 1A is a plan view of an implantable medical electrical lead, according to some embodiments of the present disclosure;
Figure 1B is an enlarged cut-away view from a proximal portion of the lead of Figure 1A, according to some embodiments;
Figure 1C is an enlarged cut-away view from an intermediate portion of the lead of Figure 1A, according to some embodiments;
Figure 1D is an enlarged cut-away view from a distal portion of the lead of Figure 1A, according to some embodiments;
Figure 1E is a plan view of an electrode assembly, which may be incorporated by the lead of Figure 1A, according to some embodiments;
Figure 1F is a plan view of another electrode assembly, which may also be incorporated by the lead of Figure 1A, according to some embodiments;
Figure 1G is a plan view of yet another electrode assembly, which may also be incorporated by the lead of Figure 1A, according to some embodiments;
Figure 2A is a longitudinal cross-section view of an assembly of an outer insulation tube and a fixation element, which may be incorporated by the lead of Figure 1A, according to some embodiments;
Figure 2B is a plan view of the fixation element, according to some embodiments;
Figure 2C is an end view of the fixation element according to some embodiments;
Figure 3 is a longitudinal cross-section view of an outer insulation tube that may be incorporated by the lead distal portion, according to some embodiments;
Figure 4A is a schematic showing the lead of Figure 1A implanted in a coronary venous system of a patient;
Figure 4B is a plan view of an exemplary delivery catheter that may be used to implant the lead as shown in Figure 4A;
Figure 5A is a plan view of an embodiment of the proximal portion of the lead of Figure 1A that incorporates a means for limiting an amount of torque transferred from a connector terminal grip sleeve of the lead to the fixation element of the lead;
Figure 5B is a longitudinal cross-section view of an outer insulation tube providing the torque limiting means of Figure 5A;
Figure 6A is a plan view of an alternate embodiment of the proximal portion of the lead of Figure 1A and an associated accessory sleeve positioned in proximity thereto for mounting around the connector terminal grip sleeve to provide an alternative means for limiting the amount of torque transferred from the connector terminal grip sleeve to the fixation element;
Figure 6B is a perspective end view of the accessory sleeve, according to some embodiments; and
Figure 6C is a plan view of the accessory sleeve mounted around the connector terminal grip sleeve, according to one or more embodiments.
Figure 7 is a flow diagram that depicts an exemplary method, not according to the invention and present for illustration purposes only, for placing a lead in a branch of the coronary sinus using a delivery device.

### DETAILED DESCRIPTION

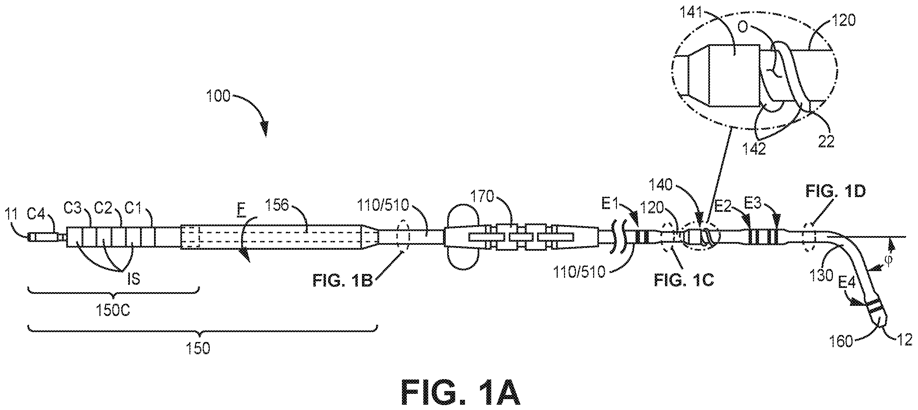

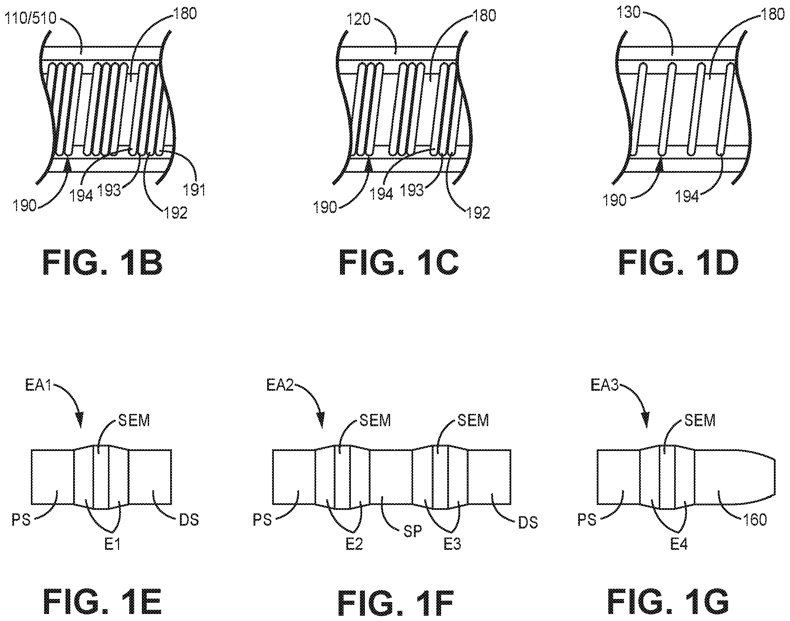

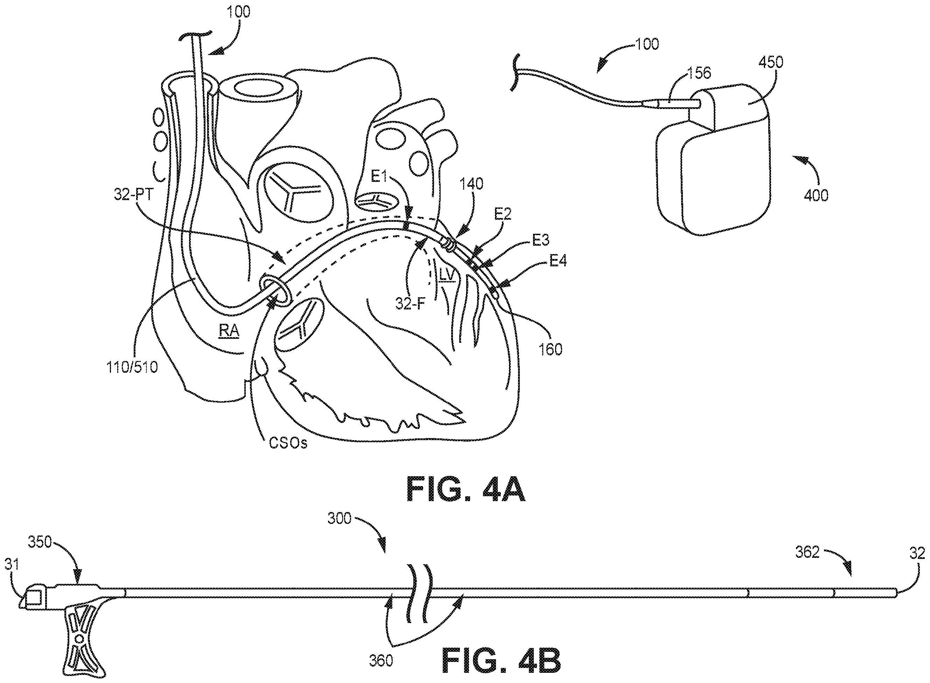

Figure 1A is a plan view of an implantable medical electrical lead 100, according to some embodiments of the present disclosure. Figure 1A illustrates lead 100 including a connector terminal 150, a plurality of electrodes E1, E2, E3, E4, and a distal-most tip 160. Electrodes E1-E4 are shown longitudinally spaced apart from one another, wherein the location of each may be suitable for delivering left heart pacing therapy when lead 100 is implanted, for example, as described below in conjunction with Figure 4A. According to an exemplary embodiment, a longitudinal spacing between first and second electrodes E1, E2 is about 21 mm, a longitudinal spacing between second and third electrodes E2, E3 is about 1.3 mm, and a longitudinal spacing between third and fourth electrodes E3, E4 is about 21 mm. Lead connector terminal 150 is shown including an assembly 150C of a plurality of contacts C1, C2, C3, C4, which are longitudinally spaced apart and electrically isolated from one another by a plurality of insulative spacers IS, and a grip sleeve 156 that extends distally from assembly 150C. Lead connector terminal 150 is configured for connection with a connector module 450 of an implantable pulse generator device 400 (Figure 4A). The construction of each of connector terminal 150 and module 450 may conform to the IS-4 standard, which is well known to those skilled in the art. Grip sleeve 156 is preferably formed from a medical grade silicone rubber. According to the illustrated embodiment, each of electrodes E1, E2, E3, E4 is electrically coupled to a corresponding connector terminal contact C1, C2, C3, C4 by a corresponding elongate conductor 190, 191, 192, 193, for example, as described below in conjunction with Figures 1A-C.

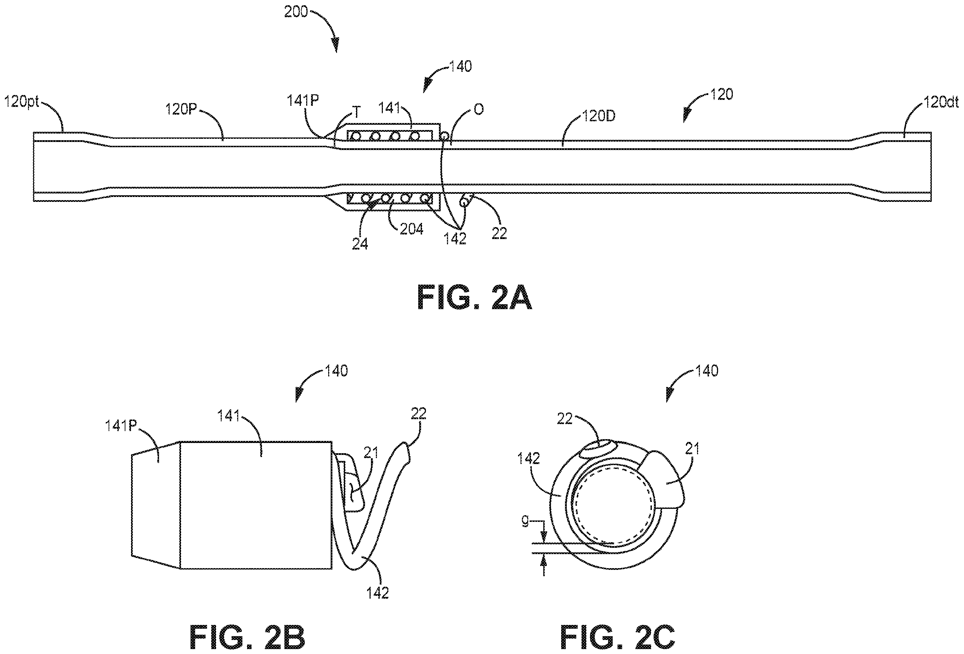

Figure 1A further illustrates lead 100 including a fixation element 140 located between a proximal-most pair of electrodes E1-E4, that is, between first electrode E1 and second electrode E2. According to the illustrated embodiment, fixation element 140 is secured to an elongate outer insulation tube 120 that extends between electrodes E1, E2 and forms an outer surface O of lead 100 therebetween. With reference to the enlarged detail view of Figure 1A, fixation element 140 is shown including a support component 141 and an elongate wire 142 wound into a helix that includes one or more exposed turns extending around and being spaced laterally from lead outer surface O. The one or more exposed turns of the helix are shown being terminated by a piercing distal tip 22 of wire 142, which is also spaced laterally from the lead outer surface O. Embodiments of fixation element 140 and outer insulation tube 120 are described in greater detail below, in conjunction with Figures 2 and 2A-B. The engagement of the helix of fixation element 140 with tissue at an implant site, for example, by applying torque at grip sleeve 156, per arrow F, to rotate lead 100, is also described in greater detail below. With further reference to Figure 1A, another elongate outer insulation tube 110 / 510 defines an outer surface, or external contour of a proximal portion of lead 100 that extends between connector terminal 150 and the proximal-most electrode E1. Figure 1A further illustrates yet another elongate outer insulation tube 130 defining an outer surface of a distal portion of lead 100 that extends in a pre-set curvature between a distal-most pair of electrodes E1-E4, that is, between third electrode E3 and fourth electrode E4. Embodiments of insulation tubes 110 / 510, 130 will be described in greater detail below. Lead 100 is also shown including a suture sleeve 170 mounted around the proximal portion thereof. The illustrated configuration of suture sleeve 170 and the employment thereof for securing the implanted lead 100 are well known to those skilled in the art.

Figures 1A-C are enlarged cut-away views taken from proximal, intermediate, and distal portions, respectively, of lead 100, as designated in Figure 1A, and according to some embodiments. In each of Figures 1B-D the corresponding outer insulation 110 / 150, 120, 130 is cut away to show an underlying multi-conductor coil 190 that includes each of the aforementioned conductors 191-194. According to the illustrated embodiment, all of conductors 191-194 extend between connector terminal 150 and first electrode E1 (Figure 1B), but, because conductor 191 is terminated at an electrical coupling with first electrode E1, only conductors 192-194 extend between first and second electrodes E1, E2 (Figure 1C), and, because each of conductors 192, 193 are terminated at an electrical coupling with the corresponding second and third electrodes E2, E3, only conductor 194 extends between third and fourth electrodes E3, E4, to be terminated at an electrical coupling with fourth electrode E4. According to an exemplary embodiment, each of conductors 191-194 is formed from medical grade MP35N alloy wire, which may have a silver core (e.g., 25%), a conductor wire composition that is well known to those skilled in the art. Furthermore, the wire of each conductor 191-194 is overlaid by a medical grade insulative coating, for example, SI-Polyimide (also known to those skilled in the art), which electrically isolates conductors 191-194 from one another. This insulative coating is removed at terminal ends of each conductor wire to electrically couple each wire to the corresponding contact C1-C4 and corresponding electrode E1-E4, for example, via a crimp, press-fit, swage, laser weld, and/or by any other suitable type of junction known in the art.

Figures 1B-D further illustrate lead 100 including an inner elongate tube 180, around which multi-conductor coil 190 extends. In an exemplary embodiment inner tube 180 may be formed from a medical grade SI-Polyimide. With reference to Figure 1A, inner tube 180 may define a lumen for passage of a guide wire and/or stylet, wherein the lumen extends from a proximal opening 11, formed in a connector terminal pin that defines contact C4. A distal opening 12 of the lumen of inner tube 180, according to some embodiments, may be formed in a medical grade silicone rubber seal mounted within distal tip 160, the configuration of which is known to those skilled in the art.

Figures 1E-G are plan views of first, second, and third electrode assemblies EA1, EA2, and EA3, respectively, according to some embodiments. Although not shown, it should be understood that each assembly EA1, EA2, EA3 has a longitudinally extending through a longitudinal bore to allow extension of the above described inner elongate tube 180 therethrough. The bore of each assembly EA1-EA3 may also allow for the electrical coupling of each conductor 191-194 to the corresponding electrode E1-E4, for example, via a press fit according to methods known in the art. Press fit is an interference fit between two parts in which one part is forced in to a smaller opening in another part. Figure 1E illustrates first electrode assembly EA1 including electrode E1, a steroid eluting member SEM mounted around an outer perimeter of electrode E1, for example, at a longitudinal midpoint thereof, and proximal and distal shanks PS, DS extending longitudinally from either end of electrode E1. Figure 1F illustrates second electrode assembly EA2 including second and third electrodes E2, E3, an insulative spacer SP extending therebetween, a pair of steroid eluting members SEM, each being mounted around an outer perimeter of the corresponding electrode E2, E3 (as in the case for first electrode E1), and proximal and distal shanks PS, DS. Figure 1G illustrates third electrode assembly EA3 including fourth electrode E4, steroid eluting member SEM mounted around an outer perimeter of fourth electrode E4 (as in the case for first, second, and third electrodes E1, E2, E3), proximal shank PS extending proximally from fourth electrode E4, and distal tip 160 extending distally from fourth electrode E4.

According to an exemplary embodiment, a bulk of each electrode E1-E4 is formed from a platinum iridium alloy, and each has a titanium nitride coating forming an exposed active surface thereof, for example, on either side of steroid eluting member SEM. Furthermore, each steroid eluting member SEM may be formed from a medical grade silicone rubber that is loaded with a steroid, for example, a 2-part enhanced tear resistant silicone rubber (dimethyl and methyl vinyl siloxane copolymers reinforced with silica) loaded with dexamethasone acetate steroid, wherein a percentage composition by weight of the steroid may range from about 25% to about 30%. Each steroid eluting member SEM may be seated in an annular groove formed around the outer perimeter of the corresponding electrode E1-E4, and bonded thereto, according to some embodiments.

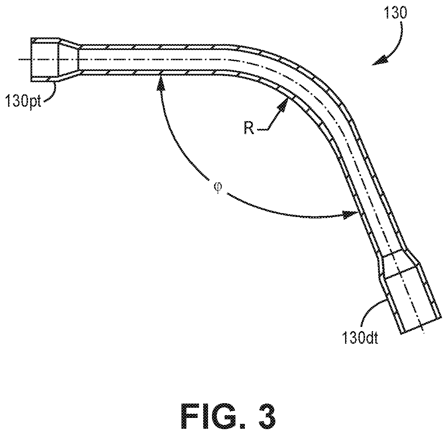

With further reference to Figures 1E-G, in conjunction with Figure 1A, proximal and distal shanks PS, DS provide means for securing outer insulation tubes 110 / 510, 120, 130 to corresponding electrodes E1-E4. For example, a distal terminal end of elongate proximal outer insulation tube 110 / 510 may be secured by thermal bonding to proximal shank PS of first electrode assembly EA1. Similarly, with additional reference to Figure 2A, proximal and distal terminal ends 120pt, 120dt of elongate outer insulation tube 120 (which may be designated as an intermediate outer insulation tube) may be secured, for example, by thermal bonding, to distal shank DS of first electrode assembly EA1 and to proximal shank PS of second electrode assembly EA2, respectively. Furthermore, with additional reference to Figure 3, proximal and distal terminal ends 130pt, 130dt of elongate distal outer insulation tube 130 may be secured, for example, by thermal bonding, to distal shank DS of second electrode assembly EA2 and to proximal shank PS of third electrode assembly EA3, respectively. According to an exemplary embodiment, each of proximal, intermediate, and distal outer insulation tubes 110 / 510, 120, 130 may each be formed from a medical grade polyurethane, for example, having a durometer of about 55D. Each of proximal and distal shanks PS, DS, insulative spacer SP, and distal tip 160 may also be formed from a medical grade polyurethane, for example, having a durometer of about 55D. According to some embodiments, the polymer portions (e.g. shanks PS, DS and spacer SP) of assemblies EA1-EA3 may be insert molded to the corresponding electrode(s) of electrodes E1-E4, according to methods known in the art.

Figure 2A is a longitudinal cross-section view of an assembly 200 of elongate intermediate outer insulation tube 120 and fixation element 140, according to some embodiments. Figure 2A illustrates the aforementioned support component 141 being mounted around tube 120 and securing the aforementioned helix/helically formed wire 142 to tube 120 by capturing a number of proximal turns 24 of the helix within a cavity 204 formed between a wall of component 141 and the outer surface of tube 120. According to an exemplary embodiment, helix wire 142 is formed from 80/20 platinum iridium alloy, support component from a medical grade polymer, such as polyurethane having a durometer of about 55D. Furthermore, cavity 204, in which proximal turns 24 are captured, may be backfilled with a medical grade adhesive. According to some preferred embodiments, piercing tip 22 is centered between the proximal and distal terminal ends 120pt, 120dt of intermediate outer insulation tube 120, and thus centered between first and second electrodes E1, E2 of lead 100. Figure 2A further illustrates intermediate outer insulation tube 120 including a proximal portion 120P having a first outer diameter, and a distal portion 120D having a second, smaller outer diameter, wherein a transition point T from the larger to the smaller diameter is approximately coincident with a tapered proximal end 141P of support component 141. According to an exemplary embodiment, the outer diameter of proximal portion 120P is about 0.051 inch (1.295 mm), and the outer diameter of distal portion 120D is about 0.045 inch (1.143 mm). The centering of piercing tip 22 between the proximal-most pair of electrodes E1, E2, in conjunction with the external contour of tube 120 has been found suitable to maintain a flexibility along this intermediate portion of lead 100. This flexibility enhances an implant operator's handling of lead 100, for example, in terms of trackability and engaging fixation element 140, when positioning and securing electrodes E1-E4 in the coronary venous system, for example, as described below in conjunction with Figure 4A.

Figures 2B-C are a plan view and an end view, respectively, of helically formed wire 142 and support component 141, according to one or more embodiments. Figures 2A-B illustrate support component 141 being formed with a rotation stop 21. Rotation stop 21 extends distally from support component cavity 204 (Figure 2B) and protrudes outward from the lead outer surface (represented by a dashed line in Figure 2C). Figure 2B further illustrates rotation stop 21 being located adjacent to a proximal-most turn of the one or more exposed turns of helix wire 142. Figure 2C further illustrates a gap g indicative of the above-described spacing between lead outer surface O (dashed line in Figure 2C) and the exposed turns of helix wire 124. Spacing g permits the exposed turns of helix wire 124 to smoothly engage with tissue at an implant site within the coronary venous system of a patient, for example, as shown in Figure 4A.

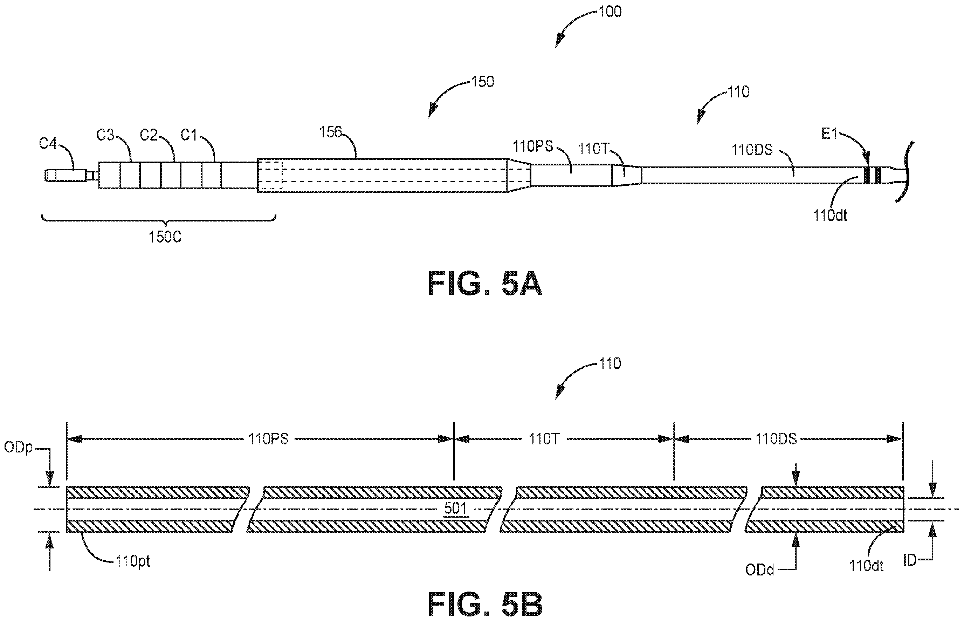

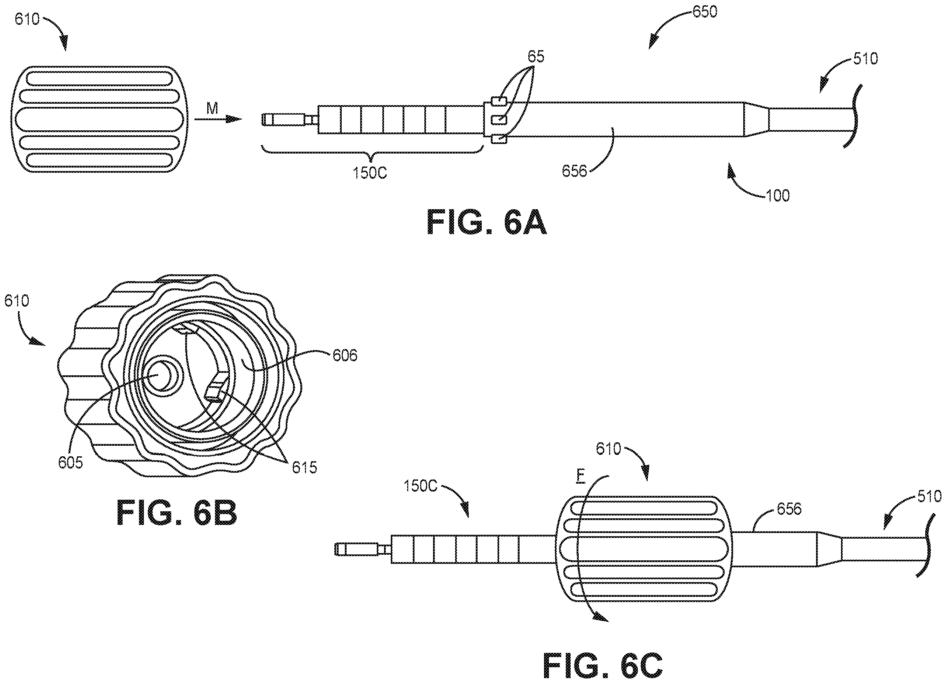

The embodiment of fixation element 140 shown in Figures 2A-C is similar to that described in the aforementioned commonly assigned United States Patent 8,755,909 to Sommer and Morgan. According to the illustrated embodiment, rotation stop 21 may prevent over-rotation of fixation element 140 that could cause tissue engaged by the exposed turns of helix wire 124 to wedge between outer insulation tube 120 (e.g., the lead outer surface) and wire 142 and support structure 141. Wedging may lead to tissue damage and/or prevent lead 100 from being withdrawn for acute repositioning or for chronic removal. As described above, torque, which is applied to connector terminal grip sleeve 156 by rotation per arrow F (Figure 1A), is transferred along lead 100 to rotate fixation member 140, the rotation of which engages tissue with piercing tip 22 of helix wire 124 at the implant site. Rotation stop 21 may provide feedback of increased resistance to this transfer of torque, when the tissue is fully engaged by helix wire 124, and thereby prevent the aforementioned over-rotation. However, in some cases when the implant operator does not detect the feedback from rotation stop 21, over-rotation may cause tissue damage and/or a permanent deformation to lead 100. According to some embodiments described below, in conjunction with Figures 5A-B and 6A-C, means for limiting the amount of torque being transferred from connector terminal grip sleeve 156 to fixation element 140 are intended to address the problem of over-rotation.

With reference back to Figure 1A, the pre-set curvature of the distal portion of lead 100 that extends between the distal-most pair of electrodes E3, E4, can further aid in the implantation of lead 100, for example, by helping to direct distal-most tip 160 toward a particular branch of the patient's coronary venous system, and in stabilizing tissue contact between fourth electrode E4 and tissue in that particular branch. According to an exemplary embodiment an angle ϕ of the pre-set curvature is about 112 degrees. Figure 3 is a longitudinal cross-section view of elongate distal outer insulation tube 130, according to some embodiments. Figure 3 illustrates proximal and distal terminal ends 130pt, 130dt of distal outer insulation tube 130 being flared for the above-described securing to distal shank DS of second electrode assembly EA2 and to proximal shank PS of third electrode assembly EA3, respectively. Figure 3 further illustrates distal outer insulation tube 130 having a single bend, at angle ϕ, pre-formed therein, so that tube 130 will impart the pre-set curvature to the lead distal portion. According to an exemplary embodiment, a radius R of the pre-formed bend may be about 0.2 inch (5.08 mm), and an OD of tube 130 between proximal and distal terminal ends 130pt, 130dt may be about 0.044 inch (1.118 mm).

Figure 4A is a schematic showing lead 100 implanted in a coronary venous system of a patient. Figure 4A illustrates implanted lead 100 being connected to a pulse generator device 400, which may be, for example, implanted in a subcutaneous pocket formed in a pectoral region of the patient. As alluded to above, electrical coupling between device 400 and contacts C1-C4 of lead connector terminal 150 (Figure 1A) for the delivery of pacing stimulation through electrodes E1-E4 is accomplished when lead connector terminal 150 (Figure 1A) is plugged into connector module 450 of device 400. Suitable configurations and constructions of device 400 are well known to those skilled in the art. Figure 4A further illustrates lead 100 passing through the right atrium RA of the patient's heart and into the coronary venous system, via an ostium of the coronary sinus CSOs, so that electrodes E1- E4 are positioned in proximity to the base of the left ventricle LV of the patient's heart at suitable locations for left heart pacing (e.g. pace the left ventricle). Figure 4B is a plan view of an exemplary delivery catheter 300 that may be used to implant lead 100 as shown in Figure 4A. Delivery catheter 300 is shown including an elongate tubular shaft 360 that defines an elongate lumen through which lead may be passed. The lumen of catheter 300 extends distally from a proximal port 31, formed within a proximal hub or handle 350, to a distal opening 32. Delivery catheter 300 may be constructed according to any suitable means known to those skilled in the art, for example, according to the specifications of the Medtronic Attain Command^{™} + SureValve^{™} Left-Heart Delivery System. Using methods known to those skilled in the art of interventional cardiology, an implant operator may introduce a distal tip 362 of catheter 300 into the patient's venous system via a subclavian stick and then pass distal tip 362 first into the right atrium RA and then into the coronary venous system through the coronary sinus ostium CSOs. Once catheter 300 is thus positioned, the operator can pass lead 100 through the catheter lumen and into the position illustrated in Figure 4A.

As described above, before lead 100 is connected to device 400, the implant operator can apply torque to lead 100, for example, at connector terminal grip sleeve 156, to rotate lead fixation element 140 so that piercing distal tip 22 of helix wire 142 engages with tissue (wall of the vein and underlying myocardial tissue) to secure lead 100 at the illustrated implant location in the coronary venous system. This operation of securing lead 100 via fixation element 140 is preferably performed while catheter distal tip 362 is still positioned within the coronary venous system, for example, with lumen distal opening 32 located in proximity to a point indicated as 32-F, just proximal to fixation element 140. Then the operator may withdraw catheter 300 so that lumen distal opening 32 is spaced some distance away from fixation element 140, for example, being located in proximity to a point indicated as 32-PT, to conduct a push test, in which a push force is applied along lead 100 from connector terminal 150. The push test can confirm the engagement of fixation element helix wire 142 for adequate securement of lead 100 at the implant site. If lead 100 is adequately secured, the proximal portion of lead 100, just proximal to first electrode E1 should buckle in response to the applied push force, the buckling being seen via fluoroscopic monitoring.

As alluded to above, over-rotation of fixation element 140 can be an issue, for example, when the implant operator does not detect the feedback provided by the above-described rotation stop 21. In some embodiments of lead 100, when an outer diameter of the proximal portion of lead 100 is greater than about 0.06 inch (1.524 mm), along an entire length thereof between connector terminal grip sleeve 156 and first electrode E1, a resulting torsional rigidity of the lead proximal portion can transfer torque from grip sleeve to fixation element 140 so efficiently that the implant operator may over-rotate before detecting the feedback from rotation stop 21. Figure 5A is a plan view of an embodiment of the proximal portion of lead 100 that incorporates a means for limiting an amount of torque transferred from connector terminal grip sleeve 156 to fixation element 140, namely an external contour of lead 100 between grip sleeve 156 and first electrode E1. Figure 5A illustrates the external contour being defined by multiple outer diameters of proximal insulation tube 110. Figure 5B is a longitudinal cross-section view of outer insulation tube 110. Figures 5A-B illustrate proximal outer insulation tube 110 including a proximal segment 110PS and a distal segment 110DS, wherein an outer diameter ODd along an overall length of distal segment 110DS is smaller than an outer diameter ODp along an overall length of proximal segment 110PS. Figures 5A-B further illustrate proximal outer insulation tube 110 including a transition segment 110T extending between proximal and distal segments 110PS, 110DS. In Figure 5B, a proximal terminal end 110pt of tube 110 is indicated, and with reference to Figure 5A, dashed lines illustrate proximal terminal end 110pt joined to assembly 150C of connector terminal contacts C1-C4, and a portion of outer insulation tube proximal segment 110PS, in proximity to proximal terminal end 110pt, extending within connector terminal grip sleeve 156. In Figure 5B, a distal terminal end 110dt of tube 110 is also indicated, and with reference to Figure 5A distal terminal end 110dt is shown secured to first electrode E1. Distal terminal end 110dt may be flared (e.g., like that shown for ends 130pt, 130dt of tube 130 in Figure 3) for securing to proximal shank PS of electrode assembly EA1, as described above in conjunction with Figure 1E. According to some exemplary embodiments, proximal outer insulation tube 110 is wholly formed from medical grade polyurethane having a durometer of about 55D, wherein outer diameter ODp along the overall length of proximal segment 110PS is in a range from 0.067 inch (1.7 mm) to 0.071 inch (1.8 mm), and outer diameter ODd along the overall length of distal segment 110DS is in a range from 0.056 inch (0.14 mm) to 0.060 inch (1.524 mm). In the exemplary embodiments, an overall length of proximal segment 110PS is in a range from 1.5 inch (3.81 cm) to seven inches (17.8 cm), preferably about three inches (7.62 cm), an overall length of transition segment 110T is preferably at least about one inch (2.54 cm), for example, being in a range from one inch (2.54 cm) to 2.5 inches (6.35 cm), and an overall length of distal segment 110DS is preferably at least twenty inches (50.8 cm), for example, being in a range from twenty inches to 28 inches (71.12 cm).

Furthermore, an inner diameter ID along an entire length of a lumen 501 of proximal outer insulation tube 110 may be about 0.034 inch (0.86 mm). According to some methods, proximal outer insulation tube 110 may be initially extruded with a single outer diameter and then subjected to an ablation process in which material is removed to form transition segment 110T and distal segment 110DS, after which tube 110 is annealed for dimensional stability. According to some methods, proximal segment 110PS may be trimmed to the aforementioned 3-inch (7.62 cm) length before annealing. Alternately, outer insulation tube 110 may be molded with the illustrated external contour.

The construction of outer insulation tube 110, as described above and illustrated in Figures 5A-B, which defines the external contour of the lead proximal portion, may limit the amount of torque transferred between grip sleeve 156 and fixation element 140 to prevent the above-described over-rotation. Furthermore, an increased flexibility in proximity to first electrode E1, provided by the smaller outer diameter ODd of distal segment 110DS can facilitate buckling in the above-described push test. Also, as was previously mentioned, connector terminal 150 may be constructed to meet a particular connector standard, for example, the IS-4 standard. The configuration of proximal outer insulation tube at the interface with connector terminal grip sleeve 156, in particular the exemplary dimensions of proximal segment PS indicated above, are significant when manufacturing lead 100 to meet such a connector standard, since these dimensions can be the same across a variety of lead configurations, thereby increasing an ease of manufacturing.

Figure 6A is a plan view of an alternate embodiment of the proximal portion of lead 100 and an associated accessory sleeve 610. According to the illustrated embodiment, a proximal outer insulation tube 510, which has a constant outer diameter in the range from 0.067 inch (1.7 mm) to 0.071 inch (1.8 mm), is substituted for the above described tube 110, so that an alternative means for limiting torque transfer to address the above-described problem of over-rotation is embodied by an interface
between accessory sleeve 610 and lead 100. Figure 6A illustrates a connector terminal 650 which may be incorporated into lead 100 in lieu of the above-described connector terminal 150. Connector terminal 650 includes the above-described contact assembly 150C, but a grip sleeve 656 of connector terminal 650 has external features 65 configured interface with internal features 615 of accessory sleeve 610, which are seen protruding from an inner surface 606 of sleeve 610 in the perspective end view of Figure 6B. Figure 6A further illustrates accessory sleeve 610 being positioned in proximity to connector terminal 650 for mounting around grip sleeve 656, per arrow M. Figure 6B further illustrates a bore 605 of accessory sleeve 610 through which connector terminal contact assembly 150C may pass to allow for the mounting, which is shown in Figure 6C. With reference to Figures 6A-C, when accessory sleeve 610 is mounted around connector terminal grip sleeve 656 and accessory sleeve 610 is rotated per arrow F, a confronting engagement between features 615 and 65 allows accessory sleeve 610 to transfer torque to lead 100 via grip sleeve 656 up to a pre-determined amount of torque, for example, up to about 0.2 to 0.4 in-oz (0.0014 - 0.0028 N-m). When the pre-determined amount of torque is reached, features 65 of grip sleeve 656, being relatively flexible compared to those of accessory sleeve 610, will deform so that accessory sleeve 610 disengages from grip sleeve 656 and moves relative thereto. According to an exemplary embodiment, grip sleeve 656 including features 65 is formed from medical grade silicone rubber, while accessory sleeve 610 including features 615 is formed from a medical grade Polyaryleheretherketone (PEEK).

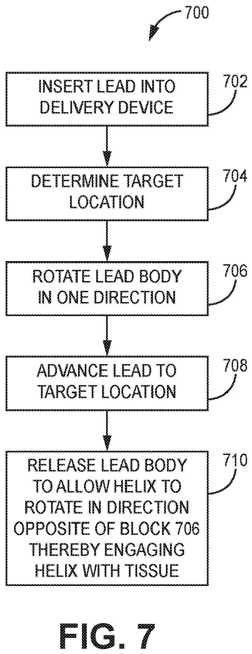

Figure 7 is a flow diagram that describes a method 700 of using a delivery system to place a medical electrical lead 100 described herein into the coronary sinus, coronary sinus branch, or coronary sinus sub-branch. Said method is not according to the invention and is present for illustration purposes only. At block 702, the physician inserts the lead 100 into a suitable left heart delivery system. According to one or more embodiments, the lead 100 is compatible with delivery systems of greater than or equal to 5.7 Fr (1.90 mm) inner diameter and guide wires of 0.36-0.46 mm (0.014-0.018 in) diameter. Different guide wires and stylets (i.e. J-stylets) can be used depending on the procedure and anatomy of the patient. At block 704, the physician may perform an occlusive venogram to determine the target vessel location. Typical target vessel locations may comprise the CS, CS branch, CS sub-branch, Great Cardiac vein, Middle Cardiac vein, basal area, or other suitable areas. At block 706, the physician may rotate lead body so that the helix rotates in one direction (e.g. counterclockwise) (CCW) through SureValve^{™} (using a transvalvular insertion tool (TVI tool) if using a self-sealing hemostasis valve). The lead body is rotated until sufficient torque has been built up in the lead body. At block 708, the physician may advance the lead to a target cardiovascular tissue location. At block 710, the lead is fixated. Fixating the lead can comprise advancing the guide wire, followed by the lead, down the target vessel. The physician rotates and holds the lead several times in a different direction than step 706 (e.g. clockwise (CW) direction).

In the foregoing detailed description, the invention has been described with reference to specific embodiments. However, it may be appreciated that various modifications and changes can be made without departing from the scope of the invention as set forth in the appended claims.

## Claims

1. An implantable medical electrical lead extending from a connector terminal (150) thereof to a distal-most tip (160) thereof, the connector terminal comprising a plurality of contacts (C1, C2, C3, C4) and a grip sleeve (156) extending distally from the contacts, and the lead further comprising:
first and second electrodes (E1, E2) both being spaced proximally from the distal-most tip and the second electrode being spaced distally from the first electrode; and
first and second elongated conductors (190, 191) electrically isolated from one another, the first and second conductors coupling the first and second electrodes, respectively, to corresponding first and second contacts of the plurality of connector terminal contacts; and
a fixation element (140) located between the first and second electrodes, the fixation element comprising a wire (142) wound into a tissue engaging helix, the helix extending around the outer surface of the lead and being terminated by a piercing distal tip of the wire, the piercing distal tip (22) spaced laterally from the lead outer surface; and
an elongated proximal outer insulation tube (110, 510) extending around the first and second elongated conductors between the connector terminal and the first electrode, the outer insulation tube comprising a proximal segment extending distally from the grip sleeve and a distal segment extending distally from the proximal segment, the distal segment having an overall length of at least 50.8 cm (20 inches), outer surfaces of each of the proximal and distal segments defining, at least in part, portions of the outer surface of the lead, the outer insulation tube transitioning from a first outer diameter along the proximal segment to a second, lesser outer diameter along the distal segment.

2. The lead of claim 1 wherein the helix further comprises one or more exposed turns extending around and being spaced laterally from an outer surface the lead, the one or more turns and being terminated by the piercing distal tip (22) of the wire.

3. The lead of any of claims 1-2 wherein the distal segment of the elongated proximal outer insulation tube extends proximally from a distal terminal end of the proximal outer insulation tube, a portion of an overall length of the proximal segment extending within the grip sleeve of the connector terminal.

4. The lead of any of claims 1-3 wherein the distal segment of the elongated proximal outer insulation tube further is configured such that the second outer diameter of the distal segment along the overall length thereof is less than the first outer diameter of the proximal segment along an overall length thereof.

5. The lead of any of claims 1-4, wherein the outer diameter of the proximal outer insulation tube proximal segment, along the overall length thereof, is in a range from 1.7 mm (0.067 inch) to 1.8 mm (0.071 inch).

6. The lead of any of claims 1-5, wherein the outer diameter of the proximal outer insulation tube distal segment along the overall length thereof is in a range from 1.4 mm (0.056 inch) to 1.524 mm (0.060 inch).

7. The lead of any of claims 1-6, wherein the proximal outer insulation tube further comprises a tapered transition segment extending from the proximal segment to the distal segment, the transition segment having an overall length of at least 2.54 cm (one inch), and a tapering outer diameter of the transition segment further defining the external contour of the lead.

8. The lead of any of claims 1-7, further comprising:
third and fourth electrodes (E3, E4) spaced distally from the first and second electrodes, the fourth electrode being located adjacent to the distal-most tip;
third and fourth elongated conductors (192, 193) electrically isolated from one another and from the first and second elongated conductors, the third and fourth conductors coupling the third and fourth electrodes, respectively, to corresponding third and fourth contacts of the plurality of connector terminal contacts; and
an elongated distal outer insulation tube (130) including a proximal terminal end secured to the third electrode and a distal terminal end secured to the fourth electrode.

9. The lead of claim 8, wherein the distal outer insulation tube has a single bend pre-formed therein.

10. The lead of any of claims 8-9, further comprising four steroid eluting members, each steroid eluting member being mounted around a corresponding electrode of the first, second, third, and fourth electrodes.

11. The lead of any of claims 1-9, further comprising a pair of steroid eluting members, each steroid eluting member being mounted around a corresponding electrode of the first and second electrodes.

12. The lead of any of claims 1-11, further comprising an elongated intermediate outer insulation tube (120) extending between the first and second electrodes and defining the outer surface of the lead around which the fixation element helix extends, the intermediate outer insulation tube including a proximal terminal end secured to the first electrode and a distal terminal end secured to the second electrode, and the fixation element being secured to the intermediate outer insulation tube so that the piercing tip thereof is centered between the proximal and distal terminal ends of the intermediate outer insulation tube.

13. The lead of claim 2, or any claim dependent thereon, further comprising a rotation stop (21) for the fixation element, the rotation stop protruding outward from the outer surface of the lead and being located adjacent to a proximal-most turn of the one or more exposed turns of the fixation element tissue engaging helix.

14. The lead of any of claims 1-13, wherein the distal terminal end of the proximal outer insulation tube is secured to the first electrode.

## Patentansprüche

1. Implantierbare medizinische elektrische Leitung, die sich von einem Verbindungsanschluss (150) davon zu einer distalsten Spitze (160) davon erstreckt, der Verbindungsanschluss umfassend eine Vielzahl von Kontakten (C1, C2, C3, C4) und eine Griffhülse (156), die sich von den Kontakten distal erstreckt, und die Leitung ferner umfassend:
erste und zweite Elektroden (E1, E2), die beide von der distalsten Spitze proximal beabstandet sind und wobei die zweite Elektrode von der ersten Elektrode distal beabstandet ist; und
einen ersten und einen zweiten länglichen Leiter (190, 191), die elektrisch voneinander isoliert sind, wobei der erste und der zweite Leiter die erste beziehungsweise die zweite Elektrode mit entsprechenden ersten und zweiten Kontakten der Vielzahl von Verbindungsanschlusskontakten koppeln; und
ein Befestigungselement (140), das sich zwischen der ersten und der zweiten Elektrode befindet, das Befestigungselement umfassend einen Draht (142), der in eine Gewebeeingriffshelix gewickelt ist, wobei sich die Helix um die Außenoberfläche der Leitung herum erstreckt und durch eine distale Durchstechspitze des Drahts begrenzt ist, wobei die distale Durchstechspitze (22) von der Leitungsaußenoberfläche seitlich beabstandet ist; und
einen länglichen proximalen äußeren Isolierschlauch (110, 510), der sich um den ersten und den zweiten länglichen Leiter herum zwischen dem Verbindungsanschluss und der ersten Elektrode erstreckt, der äußere Isolierschlauch umfassend ein proximales Segment, das sich von der Griffhülse distal erstreckt, und ein distales Segment, das sich von dem proximalen Segment distal erstreckt, wobei das distale Segment eine Gesamtlänge von mindestens 50,8 cm (20 Zoll) aufweist, wobei Außenoberflächen jedes der proximalen und der distalen Segmente mindestens teilweise Abschnitte der Außenoberfläche der Leitung definieren, wobei der äußere Isolierschlauch von einem ersten Außendurchmesser entlang des proximalen Segments zu einem zweiten, kleineren Außendurchmesser entlang des distalen Segments übergeht.

2. Leitung nach Anspruch 1, wobei die Helix ferner eine oder mehrere freiliegende Windungen umfasst, die sich um eine Außenoberfläche der Leitung herum erstrecken und von dieser seitlich beabstandet sind, wobei die eine oder die mehreren Windungen durch die distale Durchstechspitze (22) des Drahts begrenzt werden.

3. Leitung nach einem der Ansprüche 1 bis 2, wobei sich das distale Segment des länglichen proximalen äußeren Isolierschlauchs von einem distalen Anschlussende des proximalen äußeren Isolierschlauchs proximal erstreckt, wobei sich ein Abschnitt einer Gesamtlänge des proximalen Segments innerhalb der Griffhülse des Verbindungsanschlusses erstreckt.

4. Leitung nach einem der Ansprüche 1 bis 3, wobei das distale Segment des länglichen proximalen äußeren Isolierschlauchs ferner derart konfiguriert ist, dass der zweite Außendurchmesser des distalen Segments entlang der Gesamtlänge davon kleiner als der erste Außendurchmesser des proximalen Segments entlang einer Gesamtlänge davon ist.

5. Leitung nach einem der Ansprüche 1 bis 4, wobei der Außendurchmesser des proximalen Segments des proximalen äußeren Isolierschlauchs entlang der Gesamtlänge davon in einem Bereich von 1,7 mm (0,067 Zoll) bis 1,8 mm (0,071 Zoll) liegt.

6. Leitung nach einem der Ansprüche 1 bis 5, wobei der Außendurchmesser des distalen Segments des proximalen äußeren Isolierschlauchs entlang der Gesamtlänge davon in einem Bereich von 1,4 mm (0,056 Zoll) bis 1,524 mm (0,060 Zoll) liegt.

7. Leitung nach einem der Ansprüche 1 bis 6, wobei der proximale äußere Isolierschlauch ferner ein konisches Übergangssegment umfasst, das sich von dem proximalen Segment zu dem distalen Segment erstreckt, wobei das Übergangssegment eine Gesamtlänge von mindestens 2,54 cm (ein Zoll) aufweist und ein konischer Außendurchmesser des Übergangssegments ferner die externe Kontur der Leitung definiert.

8. Leitung nach einem der Ansprüche 1 bis 7, ferner umfassend:
eine dritte und eine vierte Elektrode (E3, E4), die von der ersten und der zweiten Elektrode distal beabstandet sind, wobei sich die vierte Elektrode an die distalste Spitze angrenzend befindet;
einen dritten und einen vierten länglichen Leiter (192, 193), die voneinander und von dem ersten und dem zweiten länglichen Leiter elektrisch isoliert sind, wobei der dritte und der vierte Leiter die dritte beziehungsweise die vierte Elektrode mit entsprechenden dritten und vierten Kontakten der Vielzahl von Verbindungsanschlusskontakten koppeln; und
ein länglicher distaler äußerer Isolierschlauch (130), der ein proximales Anschlussende, das an der dritten Elektrode gesichert ist, und ein distales Anschlussende einschließt, das an der vierten Elektrode gesichert ist.

9. Leitung nach Anspruch 8, wobei der distale äußere Isolierschlauch eine einzelne, darin zuvor ausgebildete Biegung aufweist.

10. Leitung nach einem der Ansprüche 8 bis 9, ferner umfassend vier Steroideluierungselemente, wobei jedes Steroideluierungselement um eine entsprechende Elektrode der ersten, der zweiten, der dritten und der vierten Elektrode herum montiert ist.

11. Leitung nach einem der Ansprüche 1 bis 9, ferner umfassend ein Paar Steroideluierungselemente, wobei jedes Steroideluierungselement um eine entsprechende Elektrode der ersten und der zweiten Elektrode herum montiert ist.

12. Leitung nach einem der Ansprüche 1 bis 11, ferner umfassend einen länglichen mittleren äußeren Isolierschlauch (120), der sich zwischen der ersten und der zweiten Elektrode erstreckt und die Außenoberfläche der Leitung, um die sich die Befestigungselementhelix erstreckt, definiert, wobei der mittlere äußere Isolierschlauch ein proximales Anschlussende, das an der ersten Elektrode gesichert ist, und ein distales Anschlussende einschließt, das an der zweiten Elektrode gesichert ist, und wobei das Befestigungselement an dem mittleren äußeren Isolierschlauch gesichert ist, sodass die Durchstechspitze davon zwischen dem proximalen und dem distalen Anschlussende des mittleren äußeren Isolierschlauchs zentriert ist.

13. Leitung nach Anspruch 2 oder einem davon abhängigen Anspruch, ferner umfassend einen Drehstopp (21) für das Befestigungselement, wobei der Drehstopp von der Außenoberfläche der Leitung nach außen vorsteht und sich an eine proximalste Windung der einen oder der mehreren freiliegenden Windungen der Befestigungselementgewebeeingriffshelix angrenzend befindet.

14. Leitung nach einem der Ansprüche 1 bis 13, wobei das distale Anschlussende des proximalen äußeren Isolierschlauchs an der ersten Elektrode gesichert ist.

## Revendications

1. Fil électrique médical implantable s'étendant d'une borne de connecteur (150) de celui-ci à une pointe la plus distale (160) de celui-ci, la borne de connecteur comprenant une pluralité de contacts (C1, C2, C3, C4) et un manchon de préhension (156) s'étendant de manière distale à partir des contacts, et le fil comprenant en outre :
des première et deuxième électrodes (E1, E2) toutes deux espacées de manière proximale de la pointe la plus distale et la deuxième électrode étant espacée de manière distale de la première électrode ; et
des premier et deuxième conducteurs allongés (190, 191) isolés électriquement l'un de l'autre, les premier et deuxième conducteurs couplant les première et deuxième électrodes, respectivement, à des premier et deuxième contacts correspondants de la pluralité de contacts de borne de connecteur ; et
un élément de fixation (140) situé entre les première et deuxième électrodes, l'élément de fixation comprenant un brin (142) enroulé en une hélice de mise en prise de tissu, l'hélice s'étendant autour de la surface externe du fil et étant terminée par une pointe distale de perforation du brin, la pointe distale de perforation (22) étant espacée latéralement de la surface externe de fil ; et
un tube d'isolation externe proximal allongé (110, 510) s'étendant autour des premier et deuxième conducteurs allongés entre la borne de connecteur et la première électrode, le tube d'isolation externe comprenant un segment proximal s'étendant de manière distale à partir du manchon de préhension et un segment distal s'étendant de manière distale à partir du segment proximal, le segment distal présentant une longueur globale d'au moins 50,8 cm (20 pouces), des surfaces externes de chacun des segments proximal et distal définissant, au moins en partie, des parties de la surface externe du fil, le tube d'isolation externe passant d'un premier diamètre externe le long du segment proximal à un second diamètre externe inférieur le long du segment distal.

2. Fil selon la revendication 1, dans lequel l'hélice comprend en outre une ou plusieurs spires exposées s'étendant autour et étant espacées latéralement d'une surface externe du fil, la ou les spires étant terminées par la pointe distale de perforation (22) du brin.

3. Fil selon l'une quelconque des revendications 1 à 2, dans lequel le segment distal du tube d'isolation externe proximal allongé s'étend de manière proximale à partir d'une extrémité terminale distale du tube d'isolation externe proximal, une partie d'une longueur globale du segment proximal s'étendant à l'intérieur du manchon de préhension de la borne de connecteur.

4. Fil selon l'une quelconque des revendications 1 à 3, dans lequel le segment distal du tube d'isolation externe proximal allongé est en outre configuré de telle sorte que le second diamètre externe du segment distal le long de sa longueur globale est inférieur au premier diamètre externe du segment proximal le long de sa longueur globale.

5. Fil selon l'une quelconque des revendications 1 à 4, dans lequel le diamètre externe du segment proximal de tube d'isolation externe proximal, le long de sa longueur globale, est compris dans une plage allant de 1,7 mm (0,067 pouce) à 1,8 mm (0,071 pouce).

6. Fil selon l'une quelconque des revendications 1 à 5, dans lequel le diamètre externe du segment distal de tube d'isolation externe proximal le long de sa longueur globale est compris dans une plage allant de 1,4 mm (0,056 pouce) à 1,524 mm (0,060 pouce).

7. Fil selon l'une quelconque des revendications 1 à 6, dans lequel le tube d'isolation externe proximal comprend en outre un segment de transition aminci s'étendant du segment proximal au segment distal, le segment de transition présentant une longueur globale d'au moins 2,54 cm (un pouce), et un diamètre externe d'amincissement du segment de transition définissant en outre le contour externe du fil.

8. Fil selon l'une quelconque des revendications 1 à 7, comprenant en outre : des troisième et quatrième électrodes (E3, E4) espacées de manière distale des première et deuxième électrodes, la quatrième électrode étant située adjacente à la pointe la plus distale ;
des troisième et quatrième conducteurs allongés (192, 193) isolés électriquement l'un de l'autre et des premier et deuxième conducteurs allongés, les troisième et quatrième conducteurs couplant les troisième et quatrième électrodes, respectivement, à des troisième et quatrième contacts correspondants de la pluralité de contacts de borne de connecteur ; et
un tube d'isolation externe distal allongé (130) comportant une extrémité terminale proximale attachée à la troisième électrode et une extrémité terminale distale attachée à la quatrième électrode.

9. Fil selon la revendication 8, dans lequel le tube d'isolation externe distal présente un coude unique qui y est préformé.

10. Fil selon l'une quelconque des revendications 8 à 9, comprenant en outre quatre éléments d'élution de stéroïde, chaque élément d'élution de stéroïde étant monté autour d'une électrode correspondante des première, deuxième, troisième et quatrième électrodes.

11. Fil selon l'une quelconque des revendications 1 à 9, comprenant en outre une paire d'éléments d'élution de stéroïde, chaque élément d'élution de stéroïde étant monté autour d'une électrode correspondante des première et deuxième électrodes.

12. Fil selon l'une quelconque des revendications 1 à 11, comprenant en outre un tube d'isolation externe intermédiaire allongé (120) s'étendant entre les première et deuxième électrodes et définissant la surface externe du fil autour duquel s'étend l'hélice de l'élément de fixation, le tube d'isolation externe intermédiaire comportant une extrémité terminale proximale attachée à la première électrode et une extrémité terminale distale attachée à la deuxième électrode, et l'élément de fixation étant attaché au tube d'isolation externe intermédiaire de sorte que sa pointe de perforation est centrée entre les extrémités terminales proximale et distale du tube d'isolation externe intermédiaire.

13. Fil selon la revendication 2, ou une quelconque revendication dépendante de celle-ci, comprenant en outre une butée de rotation (21) pour l'élément de fixation, la butée de rotation faisant saillie vers l'extérieur à partir de la surface externe du fil et étant située de manière adjacente à une spire la plus proximale de la ou des spires exposées de l'hélice de mise en prise de tissu de l'élément de fixation.

14. Fil selon l'une quelconque des revendications 1 à 13, dans lequel l'extrémité terminale distale du tube d'isolation externe proximal est attachée à la première électrode.
